# EUROPEAN PATENT APPLICATION

(11) **EP 1 138 778 A2**
(43) Date of publication of application: **04.10.2001**
(21) Application number: 01302364.3
(22) Date of filing: 14.03.2001
(51) Int. Cl.: C12Q 1/26, G01N 33/94

(54) **Methods of rapid screening of cytochrome CYP2C19 status using omeprazole**

(30) Priority: 30.03.2000 US 193100 P
(71) Applicant: Pfizer Products Inc., Groton, Connecticut 06340 (US)
(72) Inventor: Friedman, Hylar Lewis, Pfizer Global Res. and Dev., Groton, Connecticut 06340 (US); Shah, Ajit Kumar Kantilal, Pfizer Global R. and D., Groton, Connecticut 06340 (US)
(74) Representative: Hayles, James Richard

(57) **Abstract**

Mammalian subjects may be phenotyped with respect to oxidative metabolites involving CYP2C19 by measuring the ratio of omeprazole to its metabolite 5-hydroxyomeprazole in plasma samples.

## Description

### FIELD OF THE INVENTION

This invention is directed toward a method of rapidly screening subjects for poor and extensiveCYP4502C19 (CYP2C19) activity.

### BACKGROUND OF THE INVENTION

There are wide individual variations in drug efficacy and toxicity. To a great extent, these differences are the result of differences in the metabolism, distribution and elimination of the therapeutic agents. While physiological factors affecting distribution (body mass, albumin levels, etc.) and elimination (kidney function) are relatively easy to measure, the assessment of metabolic capacity is not a routine procedure. Part of this is due to the fact that metabolizing enzymes exhibit a large degree of individual variability in their levels of expression. Some polymorphic metabolic abnormalities are understood at the DNA level (genotype) but for the majority, the underlying mutations have not been identified. In such cases, metabolic phenotyping remains the only way to assess metabolic capacity through the identification of specific metabolite patterns produced by 'probe drugs'.

In probe drug phenotyping, urine or blood samples are collected before and after administration of the probe metabolized by specific enzyme(s) and responsible for the production of a particular metabolite then metabolite to parent ratios can be used to derive phenotypes. Individuals who are deficient in their ability to metabolize the probe drug are called 'slow' or 'poor' metabolizers (pm). Those who have a normal or greater than average metabolic activity are called 'fast' or 'extensive' metabolizers (em).

While the genotype prescribes the native levels of enzymatic activity, changes in the relative levels and activities of metabolizing enzymes can be produced by drug interactions and/or clinical conditions such as disease progression or malnutrition. Thus a relatively healthy patient will not necessarily have the same phenotype to a drug when that person's health has degenerated. This phenomenon has been suggested in AIDS patients for the activity of the enzyme N-acetyltransferase 2 (NAT2). In this case, the NAT2 phenotype appears to change in AIDS patients from "fast" to "slow" while the genotype remains constant.

The key barrier in the routine incorporation of metabolic activity assessments in clinical treatment is the lack of rapid, inexpensive and 'user friendly' methods for these measurements. (Ducharme, J. of Chromat. B, 678 (1996) 113-28)

M. Eickelbaum and B. Evert reviewed 5- mephenytoin polymorphism related to cytochrome P450 2C19 (CYP2C19)³.

This polymorphism was discovered by Küpfer and colleagues¹. They observed a subject who, very slowly, cleared the antiepileptic drug mephenytoin. The major metabolite is 4-hydroxy/mephenytoin, which is formed mainly from the S-enantiomer, whereas the R-enantiomer is much more slowly cleared from the body by *N*-demethylation. Poor metabolizers form only trace amounts of the hydroxylated metabolite of S-enantiomer and, as a consequence, have a much lower clearance. Phenotype assignment is now based on the ratio of S to R enantiomer, PM having a ratio of approximately 1 and EM below this value. The defect is inherited as an autosomal trait. The frequency of PM shows substantial racial differences with 2 - 3% occurring in Caucasians and up to 23% in Oriental populations². It was not until recently that CYP2C19 was identified as the enzyme that catalysed the metabolism of mephenytoin.

### References

1. Küpfer A, Desmond P, Schenker S, Branch RA. Family study of a genetically determined deficiency of mephenytoin hydroxylation in man. Pharmacogenetics 1994; 21: 173.
2. Bertilsson L. Geographical/interracial differences in polymorphic drug oxidation. Current state of the knowledge of cytochromesP450(CYP)2D6 and 2C19. Clin. Pharmacokin. 1995; 29: 192-209.
3. Clinical and Experimental Pharmacology and Physiology (1996) 23, 983-5.

### SUMMARY OF THE INVENTION

Oxidative metabolism involving the cytochrome P450 2C19 (CYP2C19) is genetically determined. This results in some individuals being phenotyped as "poor" and others as "extensive" metabolizers. Typically, the urinary molar concentration ratio of a probe drug to metabolite over an interval of 8-10 hour is determined to assign phenotype. We evaluated methods to determine CYP2C19 phenotype which may be useful to rapidly screen subjects in clinical trials using omeprazole.

In a small group of subjects our results showed that within a 4 hour plasma collection accurately predicted CYP2C19 phenotype.

This invention provides a method for phenotyping a subject as a "poor" or "extensive" metabolizer involving CYP2C19 phenotype which comprises:
a) administering a dose of omeprazole to said subject;
b) waiting for a period of time;
c) obtaining a sample of plasma from said subject;
d) measuring the concentrations of omeprazole and 5-hydroxyomeprazole in said sample;

In another aspect this invention provides a method wherein said period of time is one to four hours.

In another aspect this invention provides a method wherein said dose of omeprazole is 20 mg.

### DETAILED DESCRIPTION OF THE INVENTION

The liver contains enzymes that convert various drug substances to products, called metabolites, which can be more easily eliminated from the body, usually in the urine or feces. This conversion process, which is also known as chemical metabolism or chemical biotransformation, frequently determines the duration of action of drugs or the intensity of the drug action, which is why drugs must typically be taken several times each day to treat diseases and produce desirable pharmacological effects.

The many xenobiotic-metabolizing enzyme systems of the liver include cytochrome P450, carboxylesterases, UDP-glucuronosyltransferases, sulfotransferases, glutathione S-transferases and many others. Each of these enzyme systems is comprised of numerous individual enzymes, each of which is capable of metabolizing a wide variety of pharmaceuticals and other chemical compositions. For example, the cytochrome P450 system in the human liver is comprised of at least ten individual P450 enzymes. Of these various enzyme systems, the P450 enzymes play the most important role in determining the rate of elimination of drugs.

Metabolism by cytochrome P450 often represents the rate-limiting step in pharmaceutical elimination. Consequently, factors that lessen the activity of P450 enzymes usually prolong the effects of pharmaceuticals, whereas factors that increase cytochrome P450 activity have the opposite effect.

Changes in drug metabolism may have undesirable or toxic consequences. For example, impaired metabolism of drugs by factors that decrease cytochrome P450 activity may have the toxic effect.

Conversely, the accelerated metabolism of a drug due to increased concentrations of cytochrome P450 can also lead to a lessening of therapeutic effect.

Information from phenotyping can also be used to explain or predict adverse drug reactions that result from variances in the activity of various P450 enzymes. During phenotyping, the quantity and/or kind of metabolites produced when a drug reacts with an enzyme are used to identify the existence of that enzyme in an individual. The therapeutic or toxic effects of certain drugs can be exaggerated or compounded in a significant percentage of the population-at-large due to a genetic deficiency in a CYP P450 enzyme.

Poor metabolizers often experience exaggerated responses to drugs at dosages that are well tolerated by normal subjects who have a better capability to metabolize the same drugs. If a drug is metabolized by cytochrome CYP2C19, the drug likely will have an exaggerated or toxic effect in individuals lacking CYP2C19.

It is highly desirable to provide a method for identifying "poor" and "extensive" metabolizers of drugs. The present invention provides a simple and rapid method for identifying individuals who are "poor" and "extensive" metabolizers based on their CYP2C19 activity.

In this method, a fasting subject is dosed with an effective amount of omeprazole and the ratio of omeprazole to its metabolite 5-hydroxyomeprazole in saliva, plasma and urine is determined by high performance liquid chromatography using a reversed phase column and a fluorescence detector. A ratio of less than 7.0 defines an "extensive" metabolizer while a ratio of greater than or equal to 7.0 defines a "poor" metabolizer.

### EXAMPLE 1

Oxidative metabolism involving the cytochrome P450 2C19 (CYP2C19) is genetically determined. This results in some individuals being phenotyped as poor and others as extensive metabolizers. Typically, the plasma molar concentration ratio of a probe drug to metabolite is determined to assign phenotype. We evaluated methods to determine CYP2C19 phenotype which may be useful to rapidly screen subjects in clinical trials using omeprazole. A total of 33 healthy male subjects (age 30+/-8 year and weight 76+/-9.2 kg; 24 extensive and 9 poor metabolizers based on genotyping) were enrolled. After an overnight fast subjects received a single 20 mg dose of omeprazole (Prilosec delayed release capsule). Serial hourly plasma and saliva samples, and urine over every 2 hour interval were obtained during the 8 hour study period. Samples were assayed or omeprazole and metabolite, 5-hydroxyomeprazole, by high performance liquid chromatography and molar metabolic ratios of omeprazole to 5-hydroxyomeprazole were determined. A plasma sample within 4 hours after administration of omeprazole should be obtained to discriminate between extensive and poor metabolizers. Urine and saliva samples did not discriminate between EM and PM. The results of genotyping and phenotyping based on plasma ratios within four hours were consistent. In this small group of subjects these results suggest a plasma sample within four hours post-dose could accurately predict CYP2C19 phenotype.

## Claims

1. A method for phenotyping a mammalian subject as a "poor" or "extensive" metabolizer involving CYP2C19 phenotype which comprises:
a) administering a dose of omeprazole to said subject;
b) waiting for a period of time;
c) obtaining a sample of plasma from said subject;
d) measuring the concentrations of omeprazole and 5-hydroxyomeprazole in said sample.

2. The method of claim 1 wherein said dose of omeprazole is 20 mg.

3. The method of claim 1 wherein said period of time is one to four hours.
